# FASCICULE DE BREVET EUROPEEN

(11) **EP 1 124 983 B1**
(45) Date de publication et mention de la délivrance du brevet: **09.12.2009**
(21) Numéro de dépôt: 99950860.9
(22) Date de dépôt: 28.10.1999
(51) Int. Cl.: C12P 21/02

(54) **PROCEDE DE PRODUCTION IN VIVO DE PROTEINES CHIMIQUEMENT DIVERSIFIEES PAR INCORPORATION D'ACIDES AMINES NON CONVENTIONNELS**
IN VIVO-VERFAHREN ZUR HERSTELLUNG VON PROTEINEN, DIE NICHT-KONVENTIONELLE AMINOSÄUREN ENTHALTEN
METHOD FOR PRODUCING IN VIVO PROTEINS CHEMICALLY DIVERSIFIED BY INCORPORATING NON-STANDARD AMINO ACIDS

(30) Priorité: 28.10.1998 FR 9813533
(43) Date de publication de la demande: 22.08.2001
(73) Titulaire: INSTITUT PASTEUR, 75015 Paris (FR)
(72) Inventeur: MARLIERE, Philippe, F-91450 Etiolles (FR); DÖRING, Volker, F-75015 Paris (FR); MOOTZ, Henning, D-35037 Marburg (DE)
(74) Mandataire: Santarelli
(86) Numéro de dépôt international: PCT/FR1999/002628
(87) Numéro de publication internationale: WO 2000/024922

(56) Documents cités:
- LEMEIGNAN B. ET AL.: "Phenotypic suppression by incorporation of an alien amino acid" JOURNAL OF MOLECULAR BIOLOGY, vol. 231, no. 2, mai 1993 (1993-05), pages 161-166, XP002113371 cité dans la demande
- DÖRING V. ET AL.: "Reassigning Cysteine in the Genetic Code of Escherichia coli" GENETICS, vol. 150, no. 2, octobre 1998 (1998-10), pages 543-551, XP002113372
- BAIN J.D. ET AL.: "Ribosome-mediated incorporation of a non-standard amino acid into a peptide through expansion of the genetic code" NATURE, vol. 356, 9 avril 1992 (1992-04-09), pages 537-539, XP002113373
- IBBA M.: "STRATEGIES FOR IN VITRO AND IN VIVO TRANSLATION WITH NON-NATURAL AMINO-ACIDS" BIOTECHNOLOGY AND GENETIC ENGINEERING REVIEWS, vol. 13, 1996, pages 197-216, XP002109693 ISSN: 0264-8725

## Description

La présente invention a pour objet des méthodes permettant à des cellules procaryotes ou eucaryotes d'acquérir la capacité de produire des protéines dont les séquences d'acides aminés comprennent au moins un acide aminé non conventionnel, des méthodes de sélection desdites cellules, des procédés de production et de purification desdites protéines ainsi que les cellules et les protéines obtenues par les méthodes et procédés selon l'invention. L'invention comprend également les applications desdites cellules et protéines dans différents domaines tels que le domaine thérapeutique, cosmétique, diagnostic ou de la biosynthèse ou la biodégradation de composé organique.

Un nombre croissant de protéines produites massivement par des organismes recombinants sont employées comme catalyseurs dans l'industrie chimique ou comme agents thérapeutiques. La recherche de nouvelles protéines aux fonctions diversifiées est l'objet d'une activité intense, soit en criblant les protéines d'organismes extrêmophiles, soit en créant des variants protéiques par mutagénèse et criblage. Toutefois, la variabilité chimique des protéines qui peuvent être produites dans des organismes vivants reste limitée par l'invariance du code génétique, c'est-à-dire restreinte aux combinaisons d'un jeu canonique de 20 acides aminés. Si la descendance des espèces naturelles pouvait être progressivement remodelée dans le laboratoire de manière à adopter différents codes génétiques, l'évolution des protéines pourrait être redirigée et des sources artificielles de biodiversité ainsi établies.

La déviation expérimentale du code génétique est la seule voie qui permettrait de surmonter cette limitation. Un autre code génétique pourrait spécifier un ensemble plus ou moins grand d'acides aminés, un ensemble substitué par des monomères non canoniques ou un ensemble d'acides aminés canoniques parmi lesquels les codons sont redistribués. La spécification d'acides aminés supplémentaires dans des lignées vivantes se prêterait à de multiples applications dont la plus générique serait l'établissement d'une biodiversité artificielle.

L'incorporation, permanente ou transitoire, d'un seul acide aminé supplémentaire, portant un motif chimique qui pourrait réagir sans modifier les acides aminés conventionnels, suffirait à fonder de nouvelles méthodes de fonctionnalisation de protéine. Ceci est justement l'objet de la présente invention.

L'invention a pour objet une méthode permettant à des cellules d'acquérir la capacité de produire une protéine dont la séquence d'acides aminés comprend au moins un acide aminé non conventionnel, caractérisée en ce qu'elle comprend les étapes telles que définies dans la revendication 1.

On entendra désigner dans la présente description par le terme de protéine également les peptides ou les polypeptides, ainsi que les glycoprotéines correspondantes lorsque lesdites protéines sont glycosylées.

On entendra également désigner dans la présente description par acide aminé non conventionnel tout acide aminé autre que les acides aminés incorporés par les ribosomes au cours de la biosynthèse des protéines synthétisées par les organismes unicellulaires ou pluricellulaires procaryotes ou eucaryotes, ainsi que tout acide aminé incorporé à la place de l'acide aminé devant être normalement incorporé à cette place au regard de la séquence nucléique traduite.

On entendra également désigner dans la présente description par mutation faux-sens, une mutation qui transforme un codon qui représente un acide aminé en un codon qui code pour un autre acide aminé, ce dernier, le cas échéant, ne pouvant remplacer l'acide aminé d'origine pour donner une protéine fonctionnelle dans la protéine à la place du résidu d'acide aminé d'origine.

On entendra également désigner dans la présente description par protéine nécessaire à la croissance de cellules, une protéine qui lorsqu'elle est synthétisée par les cellules de manière fonctionnelle permet auxdites cellules de croître dans des conditions de culture données et qui lorsqu'elle est synthétisée par les cellules de manière non fonctionnelle nécessite l'introduction d'un nutriment supplémentaire dans ledit milieu de culture donné pour permettre auxdites cellules de croître. De telles protéines non fonctionnelles peuvent être par exemple synthétisées par des cellules suite à des mutations conditionnelles telles qu'une mutation de type photosensible.

Afin d'illustrer par un exemple, mais sans s'y limiter, on peut citer notamment la protéine thymidylate synthase de *E. coli* qui présente un site catalytique occupé par la cystéine au niveau de la position 146 de sa séquence d'acides aminés et dont les mutations correspondantes du gène (*thyA*) causent une exigence nutritionnelle pour la thymine ou la thymidine, aucun autre acide aminé ne pouvant remplacer la cystéine à ce site.

On entendra désigner dans la présente description par codon cible, le codon de trois bases nucléotidiques transformé par la mutation faux-sens.

L'invention comprend également une méthode selon l'invention, caractérisée en ce que le milieu de culture de l'étape c) ne contient pas le nutriment exigé par la perte de fonctionnalité de ladite protéine mutée.

Selon l'invention, l'étape c) de culture desdites cellules peut comprendre une série de cultures desdites cellules dans un milieu de culture contenant l'acide aminé codé par ledit codon cible, chacune desdites cultures de la série étant effectuée jusqu'à obtention de la phase stationnaire de croissance et suivie d'un lavage des cellules obtenues, le nombre de cultures de la série étant suffisant pour permettre la sélection de mutations augmentant la suppression de ladite mutation faux-sens dudit gène muté et la propagation de l'allèle correspondant audit gène muté.

L'invention concerne en outre une méthode selon l'invention, caractérisée en ce que la mutation faux-sens est choisie parmi les mutations faux-sens qui ne réversent spontanément qu'à une très faible fréquence, de l'ordre d'un organisme parmi au moins 10¹⁵.

De préférence, la mutation faux-sens sera choisie parmi les mutations faux-sens qui transforment un codon cible d'un gène codant pour une protéine nécessaire à la croissance de ladite cellule en un codon qui comparativement au codon cible présente un changement d'au moins deux bases, de manière plus préférée trois bases.

On préfère également les méthodes selon l'invention, caractérisées en ce que le codon cible code pour un acide aminé de faible volume stérique et/ou amphiphile et/ou de volume stérique inférieur ou sensiblement égal au volume stérique de l'acide aminé codé par la mutation faux-sens.

Parmi les codons cibles, on préfère notamment les codons cibles codant pour la cystéine et les mutations faux-sens choisies parmi les mutations faux-sens qui transforment un codon cible en un codon codant pour la valine ou l'isoleucine.

L'invention concerne en outre une méthode selon l'invention, caractérisée en ce que l'étape a) de transformation desdites cellules est réalisée au moyen d'un vecteur comprenant une séquence dudit gène codant pour une protéine nécessaire à la croissance desdites cellules comportant ladite mutation faux-sens, notamment au moyen d'un vecteur plasmidique.

De tels vecteurs seront préparés selon les méthodes couramment utilisées par l'homme du métier, et les clones en résultant peuvent être introduits dans lesdites cellules par des méthodes usuelles de recombinaison génétique telles que par exemple la lipofection, l'électroporation ou le choc thermique.

Sous un autre aspect, l'invention a pour objet une méthode de sélection de cellules capables de produire une protéine dont la séquence d'acides aminés comprend au moins un acide aminé non conventionnel caractérisée en ce qu'elle comprend les étapes a), le cas échéant b), et c) d'une méthode selon l'invention, et la sélection des cellules capables de croître à l'étape c).

De manière préférée, la méthode de sélection de cellules selon l'invention, comprendra en outre une étape d) de culture des cellules obtenues à l'étape c) dans un milieu de culture contenant ledit acide aminé codé par ledit codon cible, la concentration dudit acide aminé pouvant être à une concentration supérieure à la concentration dudit acide aminé utilisée à l'étape c), et le choix des cellules sensibles à la concentration dudit acide aminé utilisée à l'étape d).

On entend désigner par cellule sensible à un composé chimique ou biochimique ou à une concentration donnée dudit composé, une cellule dont la croissance est partiellement ou totalement inhibée lorsqu'elle est cultivée dans un milieu de culture contenant ledit composé chimique ou biochimique ou ladite concentration dudit composé.

L'invention comprend également une méthode de sélection de cellules selon l'invention, caractérisée en ce que l'aminoacyl-tRNA synthétase reconnaissant l'acide aminé codé par ladite mutation faux-sens desdites cellules sélectionnées est capable de charger sur un de ses tRNA associés un acide aminé non conventionnel ou un acide aminé autre que ledit acide aminé codé par ladite mutation faux-sens.

On entendra désigner dans la présente description par tRNA associé, un tRNA qui est reconnu par l'aminoacyl-tRNA synthétase reconnaissant un acide aminé et qui peut transférer ledit acide aminé.

L'invention comprend en outre une méthode de sélection de cellules mutantes selon l'invention, caractérisée en ce que la séquence nucléique du gène codant pour ladite aminoacyl-tRNA synthétase comporte au moins une mutation comparée à la séquence du gène sauvage correspondant, ladite mutation n'ayant pas été introduite par une technique de recombinaison génétique.

Selon un autre aspect, l'invention a pour objet les cellules procaryotes ou eucaryotes obtenues par une méthode de sélection selon l'invention.

Parmi les cellules utilisables à ces fins, on peut citer bien entendu les cellules bactériennes telles que *E. coli,* mais également les cellules de levure, de même que les cellules animales, en particulier les cultures de cellules de mammifères, comme notamment les cellules d'ovaire de hamster chinois (CHO), mais également les cellules d'insectes.

L'invention concerne également les cellules procaryotes ou eucaryotes isolées capables de produire une protéine dont la séquence d'acides aminés comprend au moins un acide aminé non conventionnel, caractérisées en ce qu'elles comprennent une aminoacyl-tRNA synthétase reconnaissant un acide aminé donné capable de charger sur un de ses tRNA associés un acide aminé non conventionnel ou un acide aminé autre que ledit acide aminé donné, et en ce que la séquence nucléique du gène codant pour ladite aminoacyl-tRNA synthétase comporte au moins une mutation comparée à la séquence du gène sauvage correspondant, ladite mutation n'ayant pas été introduite par une technique de recombinaison génétique.

Ainsi, l'invention concerne une méthode de sélection de cellules basée sur la constitution par la cellule d'une voie métabolique nécessaire à sa croissance permettant d'obtenir des cellules capables de produire un acyl-tRNA non canonique capable de charger un acide aminé non conventionnel.

Parmi les cellules selon l'invention, on préfère les cellules de bactérie, caractérisées en ce qu'elles sont choisies parmi les cellules suivantes déposées à la CNCM (Collection Nationale de Culture de Microorganismes, Paris, France) :
a) souche *E. coli* déposée à la CNCM sous le N° I-2025 le 25 mai 1998,
b) souche *E. coli* déposée à la CNCM sous le N° I-2026 le 25 mai 1998,
c) souche *E. coli* déposée à la CNCM sous le N° I-2027 le 25 mai 1998,
d) souche *E. coli* déposée à la CNCM sous le N° I-2339 le 26 octobre 1999,
e) souche E. *coli* déposée à la CNCM sous le N° I-2340 le 26 octobre 1999, et
f) souche *E. coli* déposée à la CNCM sous le N° I-2341 le 26 octobre 1999.

La souche *E. coli* K12, déposée à la CNCM sous le N° I-2025 et identifiée sous la référence β5366, est un descendant de la souche MG1655 (wt *E. coli* K12), comportant les caractéristiques suivantes :
- délétion au locus thyA et remplacement par un gène de résistance à l'érythromycine,
- porte un plasmide pTZ18 (col E1 replicon, bla⁺) avec l'allèle Cys146GUA de la thymidylate synthase.

La souche *E. coli* K12, déposée à la CNCM sous le N° I-2026 et identifiée sous la référence β8144, est un descendant de la souche MG1655 (wt *E. coli* K12), comportant les caractéristiques suivantes :
- délétion au locus thyA et remplacement par un gène de résistance à l'érythromycine,
- porte un plasmide pTZ18 (col E1 replicon, bla⁺) avec l'allèle Cys146GUA de la thymidylate synthase.

La souche *E. coli* K12, déposée à la CNCM sous le N° I-2027 et identifiée sous la référence β8146, est un descendant de la souche MG1655 5WT *E. coli* K12), comportant les caractéristiques suivantes :
- délétion au locus thyA et remplacement par un gène de résistance à l'érythromycine,
- porte un plasmide pTZ18 (col E1 replicon, bla⁺) avec l'allèle Cys146GUA de la thymidylate synthase.

La souche *E. coli* K12, déposée à la CNCM sous le N° I-2339 et identifiée sous la référence β5479, est un descendant de la souche MG1655 (wt *E. coli* K12), comportant les caractéristiques suivantes :
- délétion au locus thyA et remplacement par un gène de résistance à l'érythromycine,
- délétion au locus nrdD et remplacement par un gène de résistance à la kanamycine,
- porte l'allèle R223H du gène valS,
- porte un plasmide pTZ18 (col E1 replicon, bla⁺) avec l'allèle Cys146GUA de la thymidylate synthase.

La souche *E. coli* K12, déposée à la CNCM sous le N° I-2340 et identifiée sous la référence β5485, est un descendant de la souche MG1655 (wt *E. coli* K12), comportant les caractéristiques suivantes :
- délétion au locus thyA et remplacement par un gène de résistance à l'érythromycine,
- délétion au locus nrdD et remplacement par un gène de résistance à la kanamycine,
- porte l'allèle chromosomique Val 276 Ala du gène valS,
- porte un plasmide pTZ18 (col E1 replicon, bla⁺) avec l'allèle Cys146GUA de la thymidylate synthase.

La souche *E. coli* K12, déposée à la CNCM sous le N° I-2341 et identifiée sous la référence β5486, est un descendant de la souche MG1655 (wt *E. coli* K12) comportant les caractéristiques suivantes :
- délétion au locus thyA et remplacement par un gène de résistance à l'érythromycine,
- délétion au locus nrdD et remplacement par un gène de résistance à la kanamycine,
- porte l'allèle chromosomique Asp 230 Asn du gène valS,
- porte un plasmide pTZ18 (col E1 replicon, bla⁺) avec l'allèle Cys146GUA de la thymidylate synthase.

L'invention comprend en outre l'utilisation d'une méthode ou d'une cellule selon l'invention pour la production de protéine, notamment recombinante, dont la séquence d'acides aminés comprend au moins un acide aminé non conventionnel.

Sous un autre aspect, l'invention concerne un procédé de production d'une protéine dont la séquence d'acides aminés comprend au moins un acide aminé non conventionnel, caractérisé en ce qu'il comprend les étapes suivantes :
a) le cas échéant, la sélection d'une cellule par une méthode selon l'invention ;
b) la culture de ladite cellule sélectionnée à l'étape a) ou d'une cellule selon l'invention dans un milieu de culture et des conditions de culture permettant la croissance de ladite cellule ; et
c) l'isolement de ladite protéine comprenant au moins un acide aminé non conventionnel à partir du surnageant de culture et/ou du culot cellulaire obtenu à l'étape b).

Parmi les protéines pouvant être produites par un procédé selon l'invention, on peut mentionner, mais sans s'y limiter, les protéines qui par l'incorporation d'au moins un acide aminé non conventionnel permettent d'obtenir une activité recherchée qu'une protéine dont la séquence comporte uniquement des acides aminés conventionnels ne permet pas d'obtenir. Par activité, on entend désigner de manière générale toute activité telle qu'une activité physiologique ou biologique relative aux organismes uni- ou pluricellulaires, même partielle, comme par exemple une activité structurelle ou biochimique, par exemple enzymatique, antigénique, de type anticorps ou de modulation, de régulation ou d'inhibition d'activité biologique, ou encore telle qu'elle permette sa mise en oeuvre dans un procédé de biosynthèse ou de biodégradation de composés chimiques ou biochimiques.

On peut également mentionner parmi les protéines pouvant être produites par un procédé selon l'invention, les protéines dont l'incorporation d'au moins un acide aminé non conventionnel est effectuée de telle manière qu'il n'en résulte pas de modification approfondie de l'activité biologique de la protéine non modifiée correspondante. Outre l'activité biologique conservée de la protéine non modifiée correspondante, ces protéines selon l'invention présenteront un acide aminé non conventionnel dont les propriétés spécifiques pourront être avantageusement exploitées.

Parmi les propriétés spécifiques conférées par la présence d'un acide aminé non conventionnel, on peut citer en particulier les propriétés liées à la présence d'un groupe fonctionnel sur ledit acide aminé non conventionnel capable de réagir facilement et de manière spécifique avec un composé chimique ou biochimique dans des conditions permettant de ne pas altérer l'activité de la protéine ou évitant la modification des acides aminés conventionnels.

La présence de ce groupe fonctionnel spécifique pourra avantageusement être utilisée par exemple pour :
(i) purifier toute protéine, notamment recombinante, incorporant ledit acide aminé non conventionnel ;
(ii) coupler une telle protéine à un support solide ;
(iii) coupler à une telle protéine des molécules capables d'être détectées, telles que des sondes spectroscopiques de nature variée ;
(iv) coupler à une telle protéine des polymères lipophiles ou hydrophiles permettant de les solubiliser dans des solvants ou de faire écran à la reconnaissance par des anticorps ;
(v) coupler une telle protéine à un polynucléotide;
(vi) coupler une telle protéine à un composé chimique ou biochimique dont la présence permet d'augmenter, de diminuer, de moduler, de réguler ou de cibler l'activité biologique de ladite protéine, ou de modifier sa biodisponibilité en tant que composé à usage thérapeutique ; ou encore
(vii) fixer de manière permanente à une telle protéine un coenzyme qui sinon diffuserait en solution.

Selon la présente invention, l'incorporation d'au moins un acide aminé non conventionnel pourra porter sur des acides aminés à l'origine d'une spécificité ou de l'activité, ou à l'origine de la conformation structurale, de la charge, de l'hydrophobicité ou de la capacité de multimérisation de la protéine non modifiée correspondante. Ainsi, pourront être créées des protéines d'activité équivalente, augmentée ou diminuée, ou de spécificité équivalente, plus étroite, ou plus large que la protéine à acides aminés conventionnels non modifiée correspondante.

Par protéine non modifiée, on entend désigner la protéine sauvage ou recombinante constituée d'acides aminés conventionnels et dont est issue la protéine comprenant l'acide aminé non conventionnel.

De préférence, le procédé de production selon l'invention est caractérisé en ce que ledit milieu de culture de l'étape b) permettant la croissance de ladite cellule contient ledit acide aminé non conventionnel ou un de ses précurseurs.

Selon un mode particulier, un procédé de production selon l'invention est caractérisé en ce que ledit acide aminé non conventionnel est synthétisé par ladite cellule, la synthèse dudit acide aminé non conventionnel pouvant être augmentée par modification génétique de ladite cellule.

L'invention concerne en outre un procédé de production d'une protéine dont la séquence d'acides aminés comprend au moins un acide aminé non conventionnel selon l'invention, caractérisé en ce que ladite cellule est auxotrophe pour l'acide aminé codé par ledit codon cible.

Sont également compris dans la présente invention, les procédés selon l'invention, caractérisés en ce que ladite cellule comprend un gène d'intérêt homologue ou hétérologue dont la séquence codante comporte au moins un codon cible.

D'une manière générale, le gène d'intérêt codera pour un ARN messager qui sera ensuite traduit en protéine d'intérêt.

Le gène d'intérêt peut être isolé par toute technique conventionnelle telle que clonage, PCR (Polymerase Chain Reaction) ou encore synthétisé chimiquement. Il peut être de type génomique (muni d'un ou plusieurs introns) ou ADN complémentaire (ADNc). La protéine d'intérêt peut être constituée par une protéine mature, un précurseur et, notamment un précurseur destiné à être sécrété et comprenant un peptide signal, une protéine tronquée, une protéine chimère provenant de la fusion de séquences d'origines diverses ou encore une protéine mutée présentant des propriétés biologiques améliorées et/ou modifiées.

De manière générale, le gène d'intérêt homologue ou hétérologue pourra être choisi parmi les gènes codant pour toute protéine utilisable comme composé thérapeutique ou cosmétique, ou comme réactif de diagnostic, ou encore comme composé pouvant être mis en oeuvre dans un procédé de biosynthèse ou de biodégradation.

A titre d'exemples, on peut citer les gènes d'intérêt codant pour les protéines d'intérêt suivantes :
- cytokines ou lymphokines (interférons α, β et γ, interleukines et notamment l'IL-2, l'IL-6, l'IL-10 ou l'IL-12, facteurs nécrosant des tumeurs (TNF), facteurs stimulateurs de colonies (GM-CSF, C-CSF, M-CSF, ...) ;
- récepteurs cellulaires ou nucléaires, notamment ceux reconnus par des organismes pathogènes (virus, bactéries, ou parasites) ou leurs ligands ;
- protéines impliquées dans une maladie génétique (facteur VII, facteur VIII, facteur IX, dystrophine ou minidystrophine, insuline, protéine CFTR (Cystic Fibrosis Transmembrane Conductance Regulator), hormones de croissance (hGH) ;
- enzymes (uréase, rénine, thrombine, ...) ou toutes enzymes impliquées dans le métabolisme ou la biosynthèse des protéines, des lipides, des acides nucléiques, des sucres, des acides aminés, des acides gras ou des nucléotides ;
- inhibiteurs d'enzymes (α1-antitrypsine, antithrombine III, inhibiteurs de protéases virales, ...) ;
- composés à effet anti-tumoral capables d'inhiber au moins partiellement l'initiation ou la progression de tumeurs ou cancers (anticorps, inhibiteurs agissant au niveau de la division cellulaire ou des signaux de transduction, produits d'expression des gènes suppresseurs de tumeurs, par exemple p53 ou Rb, protéines stimulant le système immunitaire, ...) ;
- protéines du complexe majeur d'histocompatibilité des classes I ou II ou protéines régulatrices agissant sur l'expression des gènes correspondants ;
- protéines capables d'inhiber une infection virale, bactérienne ou parasitaire ou son développement (protéines antigéniques ayant des propriétés immunogènes, épitopes antigéniques, anticorps, ...) ;
- toxines telles que la ricine, toxine cholérique, diphtérique, ... ou immunotoxines ;
- marqueurs (β-galactosidase, peroxydase, ...) ; et
- luciférase, GFP (green fluorescent protein), etc..

L'invention comprend en outre un procédé de production d'une protéine selon l'invention, caractérisé en ce que le milieu de culture de l'étape b) comprend en outre les composés nécessaires à l'induction de la synthèse de la protéine codée par ledit gène d'intérêt. Ces composés sont connus de l'homme du métier et dépendent notamment de la cellule et du gène homologue ou hétérologue sélectionnés.

L'invention concerne également un procédé selon l'invention, caractérisé en ce que l'activité biologique de la protéine codée par ledit gène d'intérêt est au moins partiellement conservée après l'incorporation dudit acide aminé non conventionnel au niveau du codon cible dudit gène d'intérêt.

L'invention concerne en outre un procédé selon l'invention, caractérisé en ce que l'acide aminé non conventionnel est choisi parmi les acides aminés non conventionnels de formule I et de configuration L 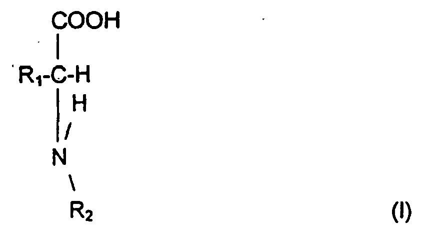 dans laquelle :
R₁ ou R₂ représente des radicaux contenant un groupe fonctionnel capable de réagir de manière sélective, de préférence choisi parmi les groupes aldéhyde, cétone, éthényle, éthynyle ou nitrile.

Parmi ces groupes, on préfère particulièrement le groupe oxo (aldéhyde ou cétone) à réactivité sélective qui faciliterait la fonctionnalisation chimique des protéines. D'autres groupes simples comme le groupe éthynyle se prêteraient également à des réactions sélectives. Un vaste corpus d'expériences conduites à l'aide de systèmes de traduction acellulaire (ex vivo) et d'acyl-tRNAs synthétisés in vitro, a démontré qu'une grande variété de groupes acyles pouvaient être transférés sur le ribosome en réponse à un codon lu par le tRNA. En bref, les modifications latérales des acides aminés semblent toutes compatibles avec la traduction (il n'a à ce jour pas été trouvé d'aminoacide dont la chaîne latérale serait assez encombrante pour bloquer la traduction) ; des substitutions du motif amino en alkyl-amino, en hydroxy et en hydrazino sont compatibles avec la chimie de transpeptidation catalysée par le ribosome (Bain et al. 1991) (il est connu que le ribosome peut former des polyesters en plus des polyamides conventionnels) ; la substitution de l'hydrogène alpha du motif H2NCH(R)-COOH par un groupe alkyle (méthyle) ou l'inversion de configuration au carbone alpha (D-amino-acides) ne sont par contre pas acceptées par le ribosome.

L'invention a également pour objet un procédé selon l'invention, pour la fonctionnalisation de protéine.

L'invention concerne également un procédé de purification de protéine, caractérisé en ce qu'il comprend les étapes suivantes :
a) l'incorporation dans la séquence d'acides aminés de ladite protéine d'un acide aminé non conventionnel contenant un groupe fonctionnel capable de réagir de manière sélective par un procédé selon l'invention ;
b) la mise en contact de la solution contenant la protéine obtenue à l'étape a) avec un support comprenant un composé capable de réagir spécifiquement avec ledit groupe fonctionnel et de fixer spécifiquement ladite protéine ; et
c) l'isolement de ladite protéine fixée sur le support.

Les procédés de purification de protéine, naturelle ou recombinante, utilisés habituellement par l'homme du métier font appel généralement à des méthodes utilisées individuellement ou en combinaison, telles que le fractionnement, les méthodes de chromatographie, les techniques d'immuno-affinité à l'aide d'anticorps mono ou polyclonaux spécifiques, etc.. Ces méthodes sont parfois longues et fastidieuses et ne permettent pas toujours d'obtenir l'activité spécifique, le taux et le rendement de purification voulus. La présence d'un groupe fonctionnel spécifique sur la protéine à purifier capable de réagir sélectivement avec le support de purification sans altérer l'activité de la protéine faciliterait grandement la purification de protéine nécessaire à leur utilisation.

L'invention concerne également un procédé de fixation d'une protéine sur un composé chimique ou biochimique, caractérisé en ce qu'il comprend les étapes suivantes :
a) l'incorporation dans la séquence d'acides aminés de ladite protéine par un procédé selon l'invention d'un acide aminé non conventionnel contenant un groupe fonctionnel capable de réagir de manière sélective ;
b) la mise en contact de la protéine obtenue à l'étape a) avec ledit composé chimique
ou biochimique comprenant un groupe capable de réagir spécifiquement avec ledit groupe fonctionnel dans un milieu permettant la réaction.

De préférence, la fixation d'une protéine sur un composé chimique ou biochimique est une fixation par liaison covalente.

Les composés chimiques ou biochimiques pouvant être utilisés dans ledit procédé de fixation selon l'invention pourront être choisis parmi tous les composés capables de réagir avec le groupe fonctionnel de l'acide aminé non conventionnel incorporé.

On entend désigner dans la présente description par complexe protéique le produit obtenu à l'étape b) du procédé décrit ci-dessus comprenant une protéine selon l'invention fixée sur un composé chimique ou biochimique.

L'invention comprend aussi un procédé selon l'invention caractérisé en ce que ledit composé chimique ou biochimique est lui-même fixé sur un support solide ou est un composé constitutif d'un support solide.

L'invention concerne en outre un procédé selon l'invention pour la préparation d'un complexe protéique.

De préférence, l'invention comprend les procédés de l'invention, caractérisés en ce que ladite protéine fixée ou ledit composé chimique ou biochimique est choisi parmi les composés thérapeutiques, cosmétiqués ou diagnostiques.

Ladite protéine fixée sera choisie en particulier parmi les protéines dont la séquence d'acides aminés comprend un acide aminé non conventionnel selon un procédé de l'invention, et dont la protéine non modifiée correspondante sauvage ou recombinante est choisie parmi les protéines utilisables comme composés thérapeutiques, cosmétiques ou comme réactifs de diagnostic.

De préférence, les procédés selon l'invention, sont caractérisés en ce que le composé chimique ou biochimique est choisi parmi les composés capables de modifier l'activité biologique de la protéine fixée.

On entend désigner par composés capables de modifier l'activité biologique d'un autre composé, un composé capable d'augmenter, de diminuer, de réguler l'activité biologique dudit autre composé.

L'invention comprend également un procédé selon l'invention, caractérisé en ce que le composé chimique ou biochimique est choisi parmi les composés dont l'activité biologique peut être modifiée par la protéine fixée.

L'invention comprend également un procédé selon l'invention, caractérisé en ce que le composé chimique ou biochimique est choisi parmi les composés comprenant une protéine, un polynucléotide, un acide gras, un sucre ou un polymère naturel ou synthétique.

Des protéines, en particulier recombinantes, et des complexes protéiques peuvent être obtenus par un procède selon l'invention concevable est également une méthode de sélection de composés capables de se lier à une proteine obtenue selon le procédé de l'invention ou capables de se lier au composé chimique ou biochimique du complexe protéique selon le procédé de l'invention. Parmi ces méthodes, on peut citer comme exemple une méthode caractérisée en ce qu'elle comprend les étapes suivantes :
a) la mise en contact dudit composé susceptible d'être sélectionné avec la protéine ou le complexe protéique obtenus selon le procédé de l'invention, ladite protéine ou complexe protéique pouvant être notamment fixée sur un support solide;
b) la détermination de la capacité dudit composé à se lier avec la protéine
ou le complexe protéique obtenus selon le procédé de l'invention.

Les composés susceptibles d'être sélectionnés peuvent être des composés organiques tels que des protéines ou hydrates de carbone ou tous autres composés organiques ou inorganiques déjà connus, ou des composés organiques nouveaux élaborés à partir de techniques de modélisation moléculaire et obtenus par synthèse chimique ou biochimique, ces techniques étant connues de l'homme de l'art.

Les cellules selon l'invention pourront également avantageusement servir de modèle et être utilisés dans des procédés pour étudier, identifier et/ou sélectionner des protéines selon l'invention ou des composés susceptibles de posséder une activité recherchée.

On peut envisager également l'utilisation d'une protéine ou d'un complexe protéique obtenus selon le procédé de l'invention comme réactif de diagnostic, ainsi que les procédés de diagnostic, notamment pour la détection, l'identification, la localisation et/ou le dosage spécifique de polypeptide ou de polynucléotide, mettant en oeuvre une protéine ou un complexe protéique selon l'invention.

Peuvent en effet être obtenus comme protéines selon les procédés décrits, des protéines ayant incorporé au moins un acide aminé non conventionnel et ayant conservé partiellement ou totalement l'activité initiale des protéines sauvages ou recombinantes non modifiées correspondantes, telles que des anticorps, des antigènes, des enzymes ou leurs fragments biologiquement actifs connus pour être utilisés dans des procédés de diagnostic.

De la même manière, peuvent être obtenus des complexes protéiques formés à partir d'une protéine obtenue selon l'invention et un composé chimique ou biochimique tels que des complexes comprenant un anticorps, un antigène ou une sonde oligonucléotidique couplé à une enzyme, à un substrat ou à une molécule capable d'être détectée.

Parmi les procédés de diagnostic envisageables, on peut citer par exemple les procédés comprenant les étapes suivantes :
a) la mise en contact de l'échantillon biologique susceptible de contenir le composé recherché avec une protéine ou un complexe protéique selon l'invention, ladite protéine ou complexe protéique pouvant être notamment fixée sur un support solide ; et
b) la mise en évidence, l'identification, la localisation et/ou le dosage du complexe formé entre le composé recherché et une protéine ou un complexe protéique selon l'invention.

L'homme du métier saura adapter avec les protéine ou les complexes protéiques obtenus selon les procédés de l'invention des procédés de diagnostic standards connus.

Les techniques et les réactifs spécifiques permettant la mise en évidence, l'identification, la localisation et/ou le dosage du complexe formé que l'on pourra utiliser dans ces procédés, sont bien connus également de l'homme du métier et sont, par exemple, les techniques ELISA, RIA, d'immunofluorescence, de PCR ou d'autres techniques d'amplification d'un acide nucléique cible connues de l'homme de l'art.

Concevable est également un kit ou nécessaire de diagnostic, notamment pour la détection, l'identification, la localisation et/ou le dosage spécifique de protéine ou de polynucléotide caractérisé en ce qu'il contient une protéine ou un complexe protéiques obtenus selon les procédés de l'invention.

Possible est en outre l'utilisation d'une, protéine, d'un complexe protéique obtenus selon les procédés de l'invention ou d'une cellule selon l'invention pour la préparation d'une composition pharmaceutique ou cosmétique.

On peut également envisager une composition pharmaceutique ou cosmétique comprenant une protéine, un complexe protéique obtenus selon les procédés de l'invention ou une cellule selon l'invention.

### EXEMPLES

### EXEMPLE 1 : Construction d'une souche d'E. coli comportant une mutation faux-sens Cys->Val au site actif de la thymidylate synthase et créant une exigence nutritionnelle pour la thymine, la thymidine ou la cystéine.

Les allèles artificiels du gène thyA sont construits par mutagénèse dirigée du plasmide pTS0 (Lemeignan et al 1993), qui dérive du plasmide pTZ18R (BioRad) par insertion du gène thyA sauvage d'*E. coli*. La mutagénèse dirigée à l'aide d'un oligonucléotide est réalisée selon la méthode décrite par Kunkel et coll. (1987) sur le phagémide pTS0. La préparation de la matrice simple brin de pTS0, amplifiée dans la souche RZ1032 (Kunkel et coll., 1987) (Hfr KL16 P045 [lysA(61-62)] dut1 ung1 thi1 relA1 supE44 zbd-279::Tn10) est réalisée selon le protocole décrit par Sambrook et coll. (1989). Un oligonucléotide phosphorylé en 5' (acheté à la société Genome Express) est utilisé comme amorce mutagène :
Oligodéoxynucléotide 1 :
   5'pTGGATAAAATGGCGCTGGCACCGGTACATGCATTCTTCCAGTTCTATGT

L'hybridation de cet oligonucléotide avec la matrice simple brin dans chacune des deux constrictions est réalisée avec 10 ng d'oligonucléotide et 0,2 µl de matrice dans un volume de 10 µl d'une solution tampon contenant 20 mM de Tris-HCl pH 7,5, 2 mM d'EDTA et 50 mM de chlorure de sodium. Les tubes sont incubés 5 mn à 70°C puis refroidis progressivement jusqu'à 30°C. A ce mélange est alors ajouté 0,5 mM de chacun des dNTP, 1 mM d'ATP, 10 mM de Tris-HCl à pH 7,5, 10 mM de chlorure de magnésium, 2 mM de dithiothréitol et 1 unité de chacune des deux enzymes du phage T4 ADN ligase et ADN polymérase. Ce mélange réactionnel d'un volume final de 20 µl est incubé 60 min à 37°C dont 5 µl sont ensuite utilisés pour transformer des cellules compétentes de la souche GT869 (Parsot, C. 1986) (thrB1004 pro thi strA hsdS lacZ ΔM15 [F'lacZ ΔM15 laclq traD36 proA+ proB+]) d'E.coli K12 suivant la méthode décrite par Sambrook et coll. (1989). Les cellules transformées sont étalées sur des boîtes de Pétri contenant du milieu LB additionné de 100 mg/l de carbénicilline. Douze clones résistant à l'antibiotique sont réisolés sur le même milieu. L'ADN simple-brin correspondant aux phagémides de ces clones est préparé et séquencé selon la méthode didéoxy (Sanger et coll., 1977). Le kit de séquençage M13 (Boehringer Mannheim, Mannheim, Allemagne) et la désoxyadénosine 5'-(α-thio)triphosphate (1300 Ci/mmol, Amersham) sont combinés selon les indications des fournisseurs. Quatre amorces sont utilisées pour déterminer la séquence des allèles de thyA :
Oligodéoxynucléotide 3 : 5'GGTGTGATCATGATGGTC
Oligodéoxynucléotide 4 : 5'CCTGCAAGATGGATTCCC
Oligodéoxynucléotide 5 : 5'CGCGCCGCATTATTGTTTC
Oligodéoxynucléotide 6 : 5'GTCTGGACCGGTGGCGACA

Le plasmide pTS1 ainsi obtenu propage l'allèle thyA:Val146, dans lequel la position 146 occupée dans le gène thyA sauvage par le codon UGC de la cystéine est occupée par le codon GUA de la valine. Le plasmide pTS1 est introduit par transformation, réalisée selon la méthode de Sambrook et coli. (1989), dans la souche ΔthyA d'*E. coli* K12, β1308 (Lemeignan et coll., 1993), dont le gène chromosomique de la thymidylate synthase, thyA, est délété. La souche transformée portant l'allèle plasmidique thyA:Val146, β5366, se montre incapable de croître sans que de la thymine ou de la thymidine soit ajoutée au milieu de culture, tout comme la souche β1308 dont elle dérive. Par contre, la souche β5366 montre une croissance marginale à 30°C sur gradient de diffusion de cystéine, réalisé dans des boîtes de Pétri contenant 25 ml de milieu minéral MS glucose à partir d'un puits central comportant 0,1 ml d'une solution 400 mM de L-cystéine. Dans les mêmes conditions, la souche β1308 ne donne lieu à aucun croissance détectable. Ainsi, la mutation faux-sens convertissant la cystéine catalytique à la position 146 de la thymidylate synthase en valine peut être partiellement supprimée par apport massif de cystéine exogène. L'addition de 0,1 mM de valine au milieu des boîtes de Pétri abolit la croissance de la souche β5366 sur gradient de cystéine. Ainsi, tout se passe comme si la cystéine pouvait s'infiltrer dans le site actif de la valyl-tRNA synthétase pour former des Cys-tRNA^{Val} erronés, aptes à corriger la substitution de la cystéine par la valine dans le site actif de la thymidylate synthase. L'excès de valine préviendrait la formation de ces Cys-tRNA^{Val} erronés.

### Exemple 2 : Construction d'une souche d'E. coli comportant une mutation faux-sens Cys->Ile au site actif de la thymidylate synthase et créant une exigence nutritionnelle pour la thymine, la thymidine ou la cystéine.

La construction correspondante est également conduite pour remplacer la cystéine en position 146 de la thymidylate synthase, par mutagénèse dirigée à l'aide de l'oligonucléotide 2, suivant le même protocole que dans l'Exemple 1.
Oligodéoxynucléotide 2 :
5'pTGGATAAAATGGCGCTGGCACCGATACATGCATTCTTCCAGTTCTATGT

Le plasmide pTS2 ainsi obtenu propage l'allèle thyA:Ile146, dans lequel la position 146 occupée dans le gène thyA sauvage par le codon UGC de la cystéine est occupée par le codon AUA de l'isoleucine. La souche propageant l'allèle plasmidique thyA:Ile146, β35274, se révèle exiger rapport nutritionnel de thymine, de thymidine ou de cystéine en excès, tout comme la souche β5366. La suppression phénotypique de la souche β5274 par la cystéine est abolie par 0,1 mM d'isoleucine, tout comme celle de la souche β5366 par la valine. Ainsi, tout se passe comme si l'isoleucyl-tRNA synthétase était capable de former des Cys-tRNA^{Ile} erronés en présence d'un excès de cystéine, et que cette formation erronée était prévenue par la présence d'un excès d'isoleucine.

### Exemple 3 : Sélection de mutants du code génétique mésincorporant la cystéine au lieu de la valine par culture sérielle en liquide et caractérisation génétique des mutants de la valyl-tRNA synthétase ainsi obtenus.

La souche β5366 portant l'allèle faux-sens thyA:Val146 sur le plasmide pTS1 est cultivée en milieu minéral MS glucose (2 g/l, Richaud et coll., 1993) supplémenté avec 0,3 mM de thymidine pendant 24 h à 30°C en aérobiose. Les cellules sont ensuite lavées deux fois avec du milieu minéral MS désoxygéné. Un milieu nutritif désoxygéné contenant 10 ml de milieu minéral MS glucose additionné de 1,5 mM de cystéine est inoculé au 1/100 à l'aide des cellules lavées. Les cellules sont ensuite cultivées en anaérobiose pendant 24 h à 30°C et un tube frais contenant 10 ml de milieu minéral MS glucose cystéine désoxygéné est inoculé avec une dilution au 1/100 de la culture en phase stationnaire précédente. Cette procédure est répétée 26 fois. A l'issue de cette propagation sérielle, 12 clones de la culture liquide sont isolés sur boîtes de milieu minéral MS glucose (2 g/l) thymidine (0,3 mM) en aérobiose et sauvegardés en suspension dans le même milieu liquide à - 80°C. Les douze clones sont testés sur des boîtes contenant du milieu minéral MS glucose additionné de facteurs nutritionnels. Tous ces clones se révèlent exiger la thymine ou la thymidine comme facteur de croissance, à moins que de la cystéine soit présente dans le milieu de culture, à une concentration d'au moins 1,5 mM.

Deux tels clones sont choisis pour une caractérisation génétique approfondie, β8144 et β8146. Des expériences de transduction par le phage P1 du caractère de résistance à la kanamycine, introduit dans le locus nrdD voisin du gène valS de la valyl-tRNA synthétase (97 mn du chromosome d'*E. coli* K12) sont effectuées à l'aide des souches β8144 et β8146. Dans les deux cas, environ la moitié des transductants résistant à la kanamycine montrent également une dépendance nutritionnelle pour la thymidine suppressible par la cystéine exogène,à la concentration d'au moins 1,5 mM. Cette proportion est en accord avec la distance génétique entre les gènes valS et nrdD (0,4 mn) et laisse supposer que le phénotype de suppression de la mutation faux-sens Cys->Val au site actif de la thymidylate synthase par de faibles concentrations de cystéine est causé par l'altération génétique du locus valS.

La fixation d'une altération génétique dans le gène valS des souches adaptées est confirmée par séquençage de ce locus : un A changé en C cause le remplacement de la lysine à la position 277 par la glutamine dans les deux souches adaptées β8144 et β8146. Le séquençage est effectué sur une matrice obtenue par réaction de polymérisation en chaîne (PCR) réalisée dans des conditions décrites par Sambrook et coll. (1989). La réaction d'amplification est réalisée dans 100. µl d'une solution contenant 10 ng d'ADN génomique des souches β8144 ou β8146, 20 pmoles de chaque amorce, 40 nmoles d'un mélange équimolaire des 4 désoxynucléotides triphosphate, 10 µl d'un tampon composé de 100 mM Tris-HCl pH 8,3, 500 mM KCI et 20 mM MgCl₂, en présence de 1 à 2 unités de Vent polymérase (Biolabs). Pour chaque réaction, 30 cycles de polymérisation sont accomplis, en utilisant un amplificateur d'ADN (Perkin-Elmer Cetus), comme suit : la dénaturation est effectuée à 94°C pendant 5 min pour le 1er cycle et 1 min pour les cycles suivant, l'hybridation à 58°C pendant 1 min et l'élongation à 72°C pendant 3 min pour les 29 premiers cycles et pendant 10 min pour le dernier cycle. Les oligonucléotides 7 et 8 sont utilisés pour l'amplification du gène.
Oligodéoxynucléotide 7 :
5'GGGGAATTCGGTGTGTGAAATTGCCGCAGAACG
Oligodéoxynucléotide 8 :
5'GGCAAGCTTCCAGTATTTCACGGGGAGTTATGC

Les fragments de PCR ainsi obtenus sont purifiés en utilisant le kit QIAquick (Qiagen) et transmis à la société Genaxis pour en déterminer la séquence.

### Exemple 4 : Suppression phénotypique par des précurseurs métaboliques de la cystéine.

L'exigence nutritionnelle en cystéine des souches adaptées β8144 et β8146 est mise à profit pour caractériser des précurseurs métaboliques qui puissent se substituer à la cystéine dans le milieu de culture sans donner lieu à la dégradation par oxydation. La S-carbamyl-L-cystéine (3 mM), la S-méthyl-L-cystéine (3 mM) et l'acide L-thiazolidine-4-carboxylate (2 mM) se révèlent capables de remplacer la cystéine comme facteur de croissance des souches adaptées β8144 et β8146, au lieu de la thymidine ou de la thymine. Les mêmes composés se révèlent capables de satisfaire l'exigence en cystéine d'un mutant cysN::kan (souche JT1, procurée par M. Berlyn, Coli Genetic Stock Center, Yale University, USA (Levh et al.,1988)). Toutefois, l'addition d'aucune de ces substances ne permet la croissance de la souche β1308 portant une délétion chromosomique du gène thyA de la thymidylate synthase, excluant ainsi leur contamination par des traces de thymine ou de thymidine.

### Exemple 5 : Sélection de mutants du code génétique mésincorporant la cystéine au lieu de la valine par isolement sur milieu solide et caractérisation génétique des mutants de la valyl-tRNA synthétase mésincorporant la cystéine.

La souche β5366 portant l'allèle faux-sens thyA:Val146 sur le plasmide pTS1 est transduite avec un lysat du phage P1 récolté sur une souche auxiliaire d'*E. coli* (β7170, Bouzon et al., 1997) dans le chromosome de laquelle un marqueur de résistance à la kanamycine avait été introduit au locus nrdD, voisin du locus valS du gène de la valyl-tRNA synthétase, produisant ainsi la souche β5419, Un allèle mutateur du gène dnaQ est introduit extemporanément par transduction de la souche β5419 à l'aide d'un lysat du phage P1 récolté sur une souche auxiliaire (MS2131, Shapiro 1990) portant un marqueur de résistance à la tétracycline dnaQ::miniTn10. Un tel clone résistant à la tétracycline et montrant un taux de mutation spontanée amplifié environ 1000 fois (pour l'acquisition de la résistance à la streptomycine) est cultivé à 30°C en milieu minimum glucose en présence de thymidine (0,3 mM). Après 24 h, les cellules sont récoltées, lavées deux fois dans un volume identique de milieu de culture sans thymidine. Un volume de 0,1 ml de la suspension résultante, correspondant à environ 10⁸ bactéries, est étalé à la surface d'une série de boîtes de Pétri contenant une concentration de S-carbamyl-L-cystéine variant entre 0 et 8 mM par incrément de 1 mM additionnant du milieu minéral MS glucose (2 g/l). La même procédure est appliquée à la souche non mutatrice β5419, au gène dnaQ sauvage. L'ensemble des boîtes de Pétri est incubé 96 h à 30°. Des colonies apparaissent sur les boîtes ayant une concentration de S-carbamyl-L-cystéine dépassant 2 mM dans le seul cas où l'allèle mutateur dnaQ::miniTn10 a été introduit dans la souche testée.

Un lysat du phage P1 récolté à partir d'un tel clone est employé pour transduire la souche β5366 portant l'allèle plasmidique thyA:Val146. Environ la moitié des transductants résistant à la kanamycine se montrent capables de croître en présence de 3 mM de S-carbamyl-L-cystéine et en absence de thymine ou de thymidine, parmi lesquels la souche β5455. L'autre moitié des transductants en est incapable et exige la thymine ou la thymidine pour proliférer, tout comme la souche β5366. Cette proportion entre les phénotypes s'accorde avec la distance génétique entre les locus valS et nrdD (0,4 mn). Ainsi, la suppression de la mutation faux-sens de thyA Cys->Val par une faible concentration de cystéine exogène pourrait résulter d'une altération du gène de la valyl-tRNA synthétase. Le locus valS d'une des souches obtenues par transduction de β5366 et capables de croître en présence de 3 mM de S-carbamyl-L-cystéine et en absence de thymine ou de thymidine, désignée β5455, est amplifié par réaction de polymérisation en chaîne et séquencé comme il est décrit dans l'Exemple 3. Un A changé en C cause le remplacement de la thréonine à la position 222 par la proline, confirmant ainsi la fixation d'une altération génétique dans le gène valS de la souche β5455.

### Exemple 6 : Sensibilité des mutants de la valyl-tRNA synthétase à des acides aminés non canoniques.

Les souches β5455, β8144 et β8146 sont testées pour leur sensibilité à des acides aminés artificiels qui présentent une ressemblance stérique avec la valine. Le test est réalisé sur des boîtes de milieu minéral MS glucose supplémenté avec de la thymidine. Les cellules sont cultivées en milieu aérobie (minéral MS glucose 0,3 mM thymidine) pendant 24 h à 30°C et diluées au 1/250 dans du milieu minéral MS. 0,5 ml de cette suspension cellulaire sont étalés sur boîtes de Pétri contenant 25 ml de milieu minéral MS glucose. Un puits est ensuite évidé au centre de la boîte et rempli avec 0,1 ml d'une solution d'un acide aminé :
(1) 100 mM L-2-amino-butyrate
(2) 100 mM L-2-amino-valérate
(3) 100 mM L-2-3-diamino-propionate
(4) 50 mM L-3-thiol-2-amino-butyrate.

Les boîtes sont ensuite incubées pendant 24 h à 30°C et l'apparition éventuelle sur les boîtes d'une zone d'inhibition autour du puits est enregistrée. Les diamètres des zones d'inhibition de croissance atténuées sur des boîtes de Pétri sont mesurés :
L-2-amino-butyrate : 5,2 cm (β5455), 5,7 cm (β8144), 6,7 cm (β8146) ;
L-2-amino-valérate : 2,1 cm (β5455), 1,5 cm (β8144), 6,7 cm (β8146) ;
L-2-3-diamino-propionate : 2,3 cm (β5455), 2,7 cm (β8144), 1,9 cm (β8146) ;
L-3-thiol-2-amino-butyrate : 2,0 cm (β5366), 4,6 cm (β5455), 4,0 cm (β8144), 4,0 cm (β8146).

Le L-2-amino-butyrate, le L-2-amino-valérate et le L-2,3 diamino-propionate aux concentrations indiquées sont sans effet sur la souche β5366 à l'allèle valS sauvage, mais inhibent la croissance des souches portant un gène valS muté. Le L-3-thiol-2-amino-butyrate inhibe la croissance de toutes les souches, mais on peut remarquer une inhibition plus importante sur les souches mutées. Ainsi, tout se passe comme si les mutants de la valyl-tRNA synthétase avaient une spécificité élargie les rendant capables de charger des tRNA^{Val} avec des acides aminés qui ne peuvent être incorporés par la forme sauvage de l'enzyme.

### Exemple 7 : Incorporation de l'acide aminé non canonique ae-aminobutyrate dans les protéines d'une souche d'E. coli mutée dans la valyl-tRNA synthétase.

Un lysat de phage P1 obtenu à partir de la souche β5455 (voir exemple 5), a été employé pour transduire la souche CU505 portant une délétion ÆilvCABD et une mutation leu la rendant auxotrophe pour la valine, l'isoleucine et la leucine. La souche CU505 a été obtenue auprès du Coli Genetic Stock Center, à Yale University (USA). Des clones transductants ont été sélectionnés sur des boîtes LB kanamycine et testés pour leur sensibilité à l'amino-butyrate (3 mM) en milieu solide MS glucose (2 g/l) contenant 0,3 mM de chacun des trois acides aminés valine, isoleucine et leucine. Environ 50 % des clones transductants ne pouvaient croître dans ces conditions, indiquant la co-transduction de l'allèle valS:T222P et le marqueur de résistance nrdD::kan (voir exemple 5). L'un des clones transductants, désigné β5498, a été utilisé pour démontrer l'incorporation d'amino-butyrate en remplacement de la valine, en comparaison avec CU505. Les deux souches ont été cultivées à 30°C en milieu liquide MS glucose (2 g/l) contenant le dipeptide Ile-Leu à la concentration de 0,3 mM et le dipeptide Ile-Val à la concentration de 0,02 mM soit en présence de 0,2 mM de L-amino-butyrate soit en absence de l'analogue. L'inoculum correspondant à chaque souche provenait d'une préculture en milieu liquide MS glucose (2 g/l) contenant le dipeptide Ile-Leu à la concentration de 0,3 mM et le dipeptide Ile-Val à la concentration de 0,04 mM. Les cultures (50 ml) en phase stationnaire après 24 h à 30°C ont été récoltées par centrifugation. Pour chaque test, le culot a ensuite été resuspendu dans 25 ml d'une solution d'acide trichloroacétique à 100 g/l (TCA 10%) à 4°C, centrifugé, resuspendu dans 5 ml de TCA 10%, centrifugé à nouveau, le culot resuspendu dans du TCA 5%, la suspension incubée à 95°C pendant 30 mn, centrifugée, le culot resuspendu dans 5 ml acétone, centrifugé, le culot resuspendu dans 5 ml acétone, centrifugé, le culot resuspendu dans 5 ml acétone, centrifugé, le culot laissé à sécher. Le résidu ainsi obtenu a été dissous dans 1 ml d'une solution de NH4HCO3 à 50 mM pour être lyophilisé. Le lyophilisat a été dissous dans 2 ml d'acide chlorhydrique 6N contenant 2 g/l de phénol, le mélange scellé dans une ampoule, puis incubé à 110°C pendant 20 h. La concentration des acides aminés de l'hydrolysat a ensuite été quantifiée par dérivatisation par la ninhydrine suivant les instructions préconisées par le fournisseur de l'analyseur Beckman 6300. L'amino-butyrate a été détecté dans l'hydrolysat des protéines seulement dans le cas où de l'amino-butyrate avait été ajouté au milieu de culture, et seulement pour la souche β5498. La proportion d'amino-butyrate remplaçait un quart de la quantité de valine, correspondant à environ 5 résidus amino-butyrate pour 100 acides aminés des protéines totales. Les résultats détaillés des analyses pour les deux souches CU505 et β5498 dans les deux conditions de culture sont donnés dans le tableau ci-après.

| **Composition chimique des protéines extraites de souches auxotrophes pour la valine et cultivées en limitation de valine, avec ou sans amino-butyrate** | | | | |
|---|---|---|---|---|
| Acide aminé incorporé dans les protéines | CU505 wt valS -Abu | β5498 valS T222P -Abu | CU505 wtvalS +Abu | β5498 valS T222P + Abu |
| Abu | 0 | 0 | 0 | 0,20 |
| Val | 0,83 | 0,79 | 0,83 | 0,61 |
| Val + Abu | 0,83 | 0,79 | 0,83 | 0,81 |
| Ala | 1,32 | 1,28 | 1,32 | 1,22 |
| Ile | 0,61 | 0,61 | 0,61 | 0,61 |

### Résultats exprimés en équivalents Leu

### Exemple 8 : Sélection de nouveaux mutants du code génétique à partir d'une souche mutatrice par isolement sur milieu solide.

La souche β5419, exprimant l'allèle inactif thyA:Val146 à partir d'un plasmide et portant le marqueur ÆnrdD::kan dans le chromosome, ainsi qu'en rend compte sa construction décrite dans l'Exemple 5, a été transduite à l'aide d'un lysat du phage P1 récolté sur la souche TAD, portant un marqueur mutateur ÆmutS::spc, conférant la résistance à la spectinomycine, en sélectionnant sur milieu solide LB contenant de la spectinomycine (25 mg/l) pour obtenir la souche β5555. Le phénotype mutateur de cette souche a été démontré en dénombrant la fréquence des mutants résistant à la rifamycine. En suivant la procédure expérimentale décrite dans l'Exemple 5, des clones capables de croître à 30°C en milieu minéral glucose sans thymidine en présence de 2 à 5 mM de S-carbamoyl-L-cystéine (SCC), ont été obtenus. Trois d'entre ces clones ont servi à préparer des lysats du phage P1, qui ont été utilisés pour transduire la souche β5366, en sélectionnant pour la résistance à la kanamycine, suivant la procédure de l'Exemple 5. Pour chacun des trois lysats, environ la moitié des transductants étaient capables de croître en milieu solide minéral glucose contenant 3 mM de SCC, indiquant la proximité d'une mutation supprimant l'allèle faux-sens thyA:C146V et du marqueur nrdD::kan. Le locus valS des trois souches β5479, β5485 et β5486, chacune correspondant à un transductant SCC-suppressible obtenu à partir d'un des trois lysats a été amplifié par PCR et séquencé comme il est décrit dans l'Exemple 3. Une mutation ponctuelle différente a été trouvée pour chacune des trois souches, à savoir Arg 223 changée en His dans la souche β5479, Val 276 changée en Ala dans la souche β5485 et Asp 230 changée en Asn dans la souche β5486. Ainsi, chaque clone présentant un phénotype de suppression du mutant faux-sens Cys 146 Val de thyA, présente également la sensibilité à l'amino-butyrate. Chacun de ces clones s'est révélé porter une mutation ponçtuelle différente dans le gène valS, validant le crible sélectif comme moyen de diversifier l'activité de la valyl-tRNA synthétase chez Escherichia coli.

### BIBLIOGRAPHIE

BAIN J.D., E.S. DIALA, C.G. GLABE, D.A. WACKER, M.H. LYTTLE, T.A. DIX and A.R. CHAMBERLIN, 1991 ; Site-specific incorporation of nonstructural residues during in vitro protein biosynthesis with semisynthetic aminoacyl-tRNAs, Biochemistry 30: 5411-5421.
BOUZON, M. and P. MARLIERE, 1997 ; Human deoxycytidine kinase as a conditional mutator in Escherichia coli. C.R. Acad.Sci. Paris 320: 427-434.
KUNKEL, T.A., and J.D. ROBERTS, 1987 ; Rapid and efficient site-specific mutagenesis.without phenotypic selection. Methods Enzymol. 154: 367-382.
LEMEIGNAN, B., P. SONIGO and P. MARLIERE, 1993 ; Phenotypic suppression by incorporation of an alien amino acid. J. Mol. Biol. 231: 161-166.
LEVH, T.F., J.C.TAYLOR and G.D. MARKHAM, 1988 ; The sulfate activation locus of Escherichia coli K12: cloning, genetic, and enzymatic characterisation. J. Biol. Chem. 263: 2409-2416.
PARSOT, C., 1986 ; Evolution of biosynthetic pathways: a common ancestor for threonine synthase, threonine dehydratase and D-serine dehydratase. EMBO J., 5: 3013-3019.
RICHAUD, C., D. MENGIN-LECREULX, S. POCHET, E.J. JOHNSON, G.N. COHEN et al., 1993 ; Directed Evolution of Biosynthetic pathways. J. Biol. Chem. 268: 26827-26835.
SAMBROOK, J., E.F. FRITSCH and T. MANIATIS, 1989 ; Molecular cloning: A Laboratory Manual. Cold Spring Harbor Laboratory Press, Cold Spring Harbor, NY.
SANGER, F., S. NICKLEN and A.R. COULSON, 1977 ; DNA sequencing with chain-terminating inhibitors. Proc. Natl. Acad. Sci. USA. 74: 5463-5467.
SHAPIRO, J.A 1990 ; Action of a transposable element in coding sequence fusions. Genetics 126: 293-299.

## Revendications

1. Méthode permettant à des cellules comprenant un gène codant pour une protéine P₀ nécessaire à la croissance desdites cellules, d'acquérir la capacité de produire une protéine dont la séquence d'acides aminés comprend au moins un acide aminé AA₁ non conventionnel, **caractérisée en ce qu'**elle comprend les étapes suivantes :
a) l'introduction dans ledit gène codant pour une protéine P₀ nécessaire à la croissance desdites cellules, d'une mutation faux-sens au niveau d'un codon-cible codant pour un acide aminé AA₀ de telle sorte que le codon muté code pour un acide aminé AA₁, ladite protéine P₁ synthétisée à partir du gène ainsi muté n'étant plus fonctionnelle ;
b) optionnellement, la culture des cellules obtenues à l'étape a) dans lesquelles la mutation faux-sens a été introduite, dans un milieu de culture contenant un nutriment compensant la perte de fonctionnalité de ladite protéine P₁ ainsi mutée; et
c) la culture des cellules obtenues à l'étape a) ou à l'étape b), dans un milieu de culture contenant l'acide aminé AA₀ codé par ledit codon-cible avant introduction de la mutation faux-sens.

2. Méthode selon la revendication 1 , **caractérisée en ce que** l'étape c) de culture desdites cellules comprend une série de cultures desdites cellules dans un milieu de culture contenant l'acide aminé codé par ledit codon cible, chacune desdites cultures de la série étant effectuée jusqu'à obtention de la phase stationnaire de croissance et suivie d'un lavage des cellules obtenues, le nombre de cultures de la série étant suffisant pour permettre la sélection de mutations augmentant la suppression de ladite mutation faux-sens dudit gène muté.

3. Méthode selon l'une des revendications 1 et 2, **caractérisée en ce que** la mutation faux-sens est choisie parmi les mutations faux-sens qui ne réversent spontanément qu'à une très faible fréquence, de l'ordre d'un organisme parmi au moins 10¹⁵.

4. Méthode selon l'une des revendications 1 à 3, **caractérisée en ce que** la mutation faux-sens transforme un codon cible d'un gène codant pour une protéine nécessaire à la croissance de ladite cellule en un codon qui comparativement au codon cible présente un changement d'au moins deux bases, de préférence trois bases.

5. Méthode selon l'une des revendications 1 à 4, **caractérisée en ce que** le codon cible code pour un acide aminé de faible volume stérique.

6. Méthode selon l'une des revendications 1 à 5, **caractérisée en ce que** le codon cible code pour un acide aminé amphiphile.

7. Méthode selon l'une des revendications 1 à 6, **caractérisée en ce que** le codon cible code pour un acide aminé dont le volume stérique est inférieur ou égal au volume stérique de l'acide aminé codé par la mutation faux-sens.

8. Méthode selon l'une des revendications 4 à 7, **caractérisée en ce que** le codon cible code pour la cystéine.

9. Méthode selon l'une des revendications 4 à 8, **caractérisée en ce que** l'acide aminé codé par la mutation faux-sens est la valine ou l'isoleucine.

10. Méthode selon l'une des revendications 1 à 9, **caractérisée en ce que** l'étape a) de transformation desdites cellules est réalisée au moyen d'un vecteur comprenant une séquence dudit gène codant pour une protéine nécessaire à la croissance desdites cellules comportant ladite mutation faux-sens.

11. Méthode selon la revendication 10, **caractérisée en ce que** ledit vecteur est un vecteur plasmidique.

12. Méthode de sélection de cellules capables de produire une protéine dont la séquence d'acides aminés comprend au moins un acide aminé non conventionnel **caractérisée en ce qu'**elle comprend les étapes a), le cas échéant b), et c) d'une méthode selon l'une des revendications 1 à 12, et la sélection des cellules capables de croître à l'étape c).

13. Méthode de sélection de cellules selon la revendication 12, **caractérisée en ce qu'**elle comprend en outre une étape d) de culture des cellules obtenues à l'étape c) dans un milieu de culture contenant ledit acide aminé codé par ledit codon cible, la concentration dudit acide aminé pouvant être à une concentration supérieure à la concentration dudit acide aminé utilisée à l'étape c), et le choix des cellules sensibles à la concentration dudit acide aminé utilisée à l'étape d).

14. Méthode de sélection de cellules selon l'une des revendications 12 et 13, **caractérisée en ce que** l'aminoacyl-tRNA synthétase reconnaissant l'acide aminé codé par ladite mutation faux-sens desdites cellules sélectionnées est capable de charger sur un de ses tRNA associés un acide aminé non conventionnel ou un acide aminé autre que ledit acide aminé codé par ladite mutation faux-sens.

15. Méthode de sélection de cellules selon la revendication 14, **caractérisée en ce que** la séquence nucléique du gène codant pour ladite aminoacyl-tRNA synthétase comporte au moins une mutation comparée à la séquence du gène sauvage correspondant.

16. Méthode de sélection de cellules selon la revendication 15, **caractérisée en ce que** ladite mutation n'a pas été introduite par une technique de recombinaison génétique.

17. Cellule susceptible d'être obtenue par une méthode selon l'une quelconque des revendications 12 à 16.

18. Cellule isolée capable de produire une protéine dont la séquence d'acides aminés comprend au moins un acide aminé non conventionnel, **caractérisée en ce qu'**elle comprend une aminoacyl-tRNA synthétase reconnaissant un acide aminé donné capable de charger sur un de ses tRNA associés un acide aminé non conventionnel ou un acide aminé autre que ledit acide aminé donné, et **en ce que** la séquence nucléique du gène codant pour ladite aminoacyl-tRNA synthétase comporte au moins une mutation comparée à la séquence du gène sauvage correspondant, ladite mutation n'ayant pas été introduite par une technique de recombinaison génétique.

19. Cellule selon la revendication 17 ou 18, **caractérisée en ce qu'**il s'agit d'une cellule procaryote ou eucaryote.

20. Cellule selon la revendication 19, **caractérisée en ce qu'**il s'agit d'une cellule procaryote.

21. Cellule selon l'une des revendications 17 à 20, **caractérisée en ce qu'**elle est choisie parmi les cellules suivantes déposées à la CNCM (Collection Nationale de Culture de Microorganismes, Paris, France) :
a) souche *E. coli* déposée à la CNCM sous le N° I-2025 le 25 mai 1998,
b) souche *E. coli* déposée à la CNCM sous le N° I-2026 le 25 mai 1998, et
c) souche *E. coli* déposée à la CNCM sous le N° I-2027 le 25 mai 1998.
d) souche *E. coli* déposée à la CNCM sous le N° I-2339 le 26 octobre 1999,
e) souche *E. coli* déposée à la CNCM sous le N° I-2340 le 26 octobre 1999, et
f) souche *E. coli* déposée à la CNCM sous le N° I-2341 le 26 octobre 1999.

22. Utilisation d'une méthode selon l'une des revendications 1 à 16 pour la production de protéine dont la séquence d'acides aminés comprend au moins un acide aminé non conventionnel.

23. Utilisation d'une cellule selon l'une des revendications 17 à 21 pour la production de protéine dont la séquence d'acides aminés comprend au moins un acide aminé non conventionnel.

24. Procédé de production d'une protéine dont la séquence d'acides aminés comprend au moins un acide aminé non conventionnel, **caractérisé en ce qu'**il comprend les étapes suivantes :
a) le cas échéant, la sélection d'une cellule par une méthode selon l'une des revendications 12 à 16 ;
b) la culture de ladite cellule sélectionnée à l'étape a) ou d'une cellule selon l'une des revendications 17 à 21 dans un milieu de culture et des conditions de culture permettant la croissance de ladite cellule ; et
c) l'isolement de ladite protéine comprenant au moins un acide aminé non conventionnel à partir du surnageant de culture et/ou du culot cellulaire obtenu à l'étape b).

25. Procédé selon la revendication 24, **caractérisé en ce que** ledit milieu de culture de l'étape b) permettant la croissance de ladite cellule contient ledit acide aminé non conventionnel ou un de ses précurseurs.

26. Procédé selon la revendication 24, **caractérisé en ce que** ledit acide aminé non conventionnel est synthétisé par ladite cellule.

27. Procédé selon la revendication 26, **caractérisé en ce que** la synthèse dudit acide aminé non conventionnel est augmentée par modification génétique de ladite cellule.

28. Procédé selon l'une des revendications 24 à 27, **caractérisé en ce que** ladite cellule est auxotrophe pour l'acide aminé codé par ledit codon cible.

29. Procédé selon l'une des revendications 24 à 28, **caractérisé en ce que** ladite cellule comprend un gène homologue ou hétérologue d'intérêt dont la séquence codante comporte au moins un codon cible.

30. Procédé selon la revendication 29, **caractérisé en ce** l'étape b) comprend les composés nécessaires à l'induction de la synthèse de la protéine codée par ledit gène d'intérêt.

31. Procédé selon la revendication 29 ou 30, **caractérisé en ce que** l'activité biologique de la protéine codée par ledit gène d'intérêt est au moins partiellement conservée après l'incorporation dudit acide aminé non conventionnel au niveau du codon cible dudit gène d'intérêt.

32. Procédé selon l'une des revendications 24 à 31, **caractérisé en ce que** l'acide aminé non conventionnel est choisi parmi les acides aminés non conventionnels de formule I de configuration L 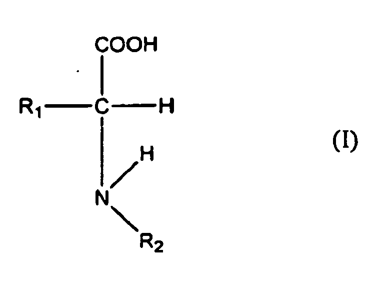 dans laquelle :
R₁ ou R₂ représente des radicaux contenant un groupe fonctionnel capable de réagir de manière sélective avec un composé chimique ou biochimique.

33. Procédé selon la revendication 32, **caractérisé en ce que** le groupe fonctionnel est choisi parmi les groupes aldéhyde, cétone, éthényle, éthynyle ou nitrile.

34. Procédé selon l'une des revendications 24 à 33, pour la fonctionnalisation d'une protéine.

35. Procédé de purification de protéine, **caractérisé en ce qu'**il comprend les étapes suivantes :
a) l'incorporation dans la séquence d'acides aminés de ladite protéine d'un acide aminé non conventionnel contenant un groupe fonctionnel capable de réagir de manière sélective par un procédé selon l'une des revendications 24 à 34 ;
b) la mise en contact de la solution contenant la protéine obtenue à l'étape a) avec un support comprenant un composé capable de réagir spécifiquement avec ledit groupe fonctionnel et de fixer spécifiquement ladite protéine ; et
c) l'isolement de ladite protéine fixée sur le support.

36. Procédé de fixation d'une protéine sur un composé chimique ou biochimique, **caractérisé en ce qu'**il comprend les étapes suivantes :
a) l'incorporation dans la séquence d'acides aminés de ladite protéine par un procédé selon l'une des revendications 24 à 34 d'un acide aminé non conventionnel contenant un groupe fonctionnel capable de réagir de manière sélective ;
b) la mise en contact de la protéine obtenue à l'étape a) avec ledit composé chimique ou biochimique comprenant un groupe capable de réagir spécifiquement avec ledit groupe fonctionnel dans un milieu permettant la réaction.

37. Procédé selon la revendication 36, **caractérisé en ce que** ledit composé chimique ou biochimique est lui-même fixé sur un support solide ou est un composé constitutif d'un support solide.

38. Procédé selon la revendication 36 pour la préparation d'un complexe protéique.

39. Procédé selon la revendication 38, **caractérisé en ce que** la protéine fixée ou le composé chimique ou biochimique est choisi parmi les composés thérapeutiques, cosmétiques ou diagnostiques.

40. Procédé selon la revendication 38 ou 39, **caractérisé en ce que** le composé chimique ou biochimique est choisi parmi les composés capables de modifier l'activité biologique de la protéine fixée.

41. Procédé selon la revendication 38 ou 39, **caractérisé en ce que** le composé chimique ou biochimique est choisi parmi les composés dont l'activité biologique peut être modifiée par la protéine fixée.

42. Procédé selon l'une des revendications 38 à 41, **caractérisé en ce que** le composé chimique ou biochimique est choisi parmi les composés comprenant une protéine, un polynucléotide, un acide gras, un sucre ou un polymère naturel ou synthétique.

## Claims

1. Method which allows cells comprising a gene encoding a protein P₀ necessary for the growth of said cells, to acquire the capacity to produce a protein the amino acid sequence of which comprises at least one unconventional amino acid AA₁, **characterized in that** it comprises the following steps,
a) introducing into said gene encoding a protein P₀ necessary for the growth of said cells, of a missense mutation in a target codon encoding amino acid AA₀, such that the mutated codon encodes an amino acid AA₁, said protein P₁ synthesized from the gene thus mutated no longer being functional;
b) optionally, culturing the cells obtained in step a) in which cells the missense mutation was introduced, in a culture medium containing a nutrient compensating the loss of functionality of said protein P₁ thus mutated; and
c) culturing the cells obtained in step a) or step b), in a culture medium containing the amino acid AA₀ encoded by said target codon before introducing the missense mutation.

2. Method according to claim 1, **characterized in that** step c) of culturing said cells comprises a series of cultures of said cells in a culture medium containing the amino acid encoded by said target codon, each of said cultures of the series being carried out until the stationary growth phase is obtained, and followed by washing of the cells obtained, the number of cultures of the series being sufficient to allow the selection of mutations which increase the suppression of said missense mutation of said mutated gene.

3. Method according to one of claims 1 and 2, **characterized in that** the missense mutation is chosen from the missense mutations which reverse spontaneously only at a very low frequency, of the order of one organism from at least 10¹⁵.

4. Method according to one of claims 1 to 3, **characterized in that** the missense mutation transforms a target codon of a gene encoding a protein required for the growth of said cell into a codon which, in comparison with the target codon, exhibits a change of at least two bases, more preferably three bases.

5. Method according to one of claims 1 to 4, **characterized in that** the target codon encodes an amino acid of small stearic volume.

6. Method according to one of claims 1 to 5, **characterized in that** the target codon encodes an amphiphilic amino acid.

7. Method according to one of claims 1 to 6, **characterized in that** the target codon encodes an amino acid the stearic volume of which is less than, or equal to, the stearic volume of the amino acid encoded by the missense mutation.

8. Method according to one of claims 4 to 7, **characterized in that** the target codon encodes cysteine.

9. Method according to one of claims 4 to 8, **characterized in that** the amino acid encoded by the missense mutation is valine or isoleucine.

10. Method according to one of claims 1 to 9, **characterized in that** step a) for transforming said cells is carried out using a vector comprising a sequence of said gene encoding a protein required for the growth of said cells comprising said missense mutation.

11. Method according to claim 10, **characterized in that** said vector is a plasmid vector.

12. Method for selecting cells capable of producing a protein the amino acid sequence of which comprises at least one unconventional amino acid, **characterized in that** it comprises steps a), where appropriate b), and c) of a method according to one of claims 1 to 12, and the selection of cells capable of growing in step c).

13. Method for selecting cells according to claim 12, **characterized in that** it comprises moreover a step d) for culturing the cells in step c) in a culture medium containing said amino acid encoded by said target codon, the concentration of said amino acid possibly being at a concentration higher than the concentration of said amino acid used in step c), and the choice of cells sensitive to the concentration of said amino acid used in step d).

14. Method for selecting cells according to one of claims 12 and 13, **characterized in that** the aminoacyl-tRNA synthetase which recognizes the amino acid encoded by said missense mutation of said selected cells is capable of charging onto one of its associated tRNAs an unconventional amino acid or an amino acid other than said amino acid encoded by said missense mutation.

15. Method for selecting cells according to claim 14, **characterized in that** the nucleic acid sequence of the gene encoding said aminoacyl-tRNA synthetase includes at least one mutation compared with the sequence of the corresponding wild-type gene.

16. Method for selecting cells according to claim 15, **characterized in that** said mutation was not introduced by a genetic recombination technique.

17. Cell capable of being obtained by a method according to any one of claims 12 to 16.

18. Isolated cell capable of producing a protein the amino acid sequence of which comprises at least one unconventional amino acid, **characterized in that** it comprises an aminoacyl-tRNA synthetase which recognizes a given amino acid and which is capable of charging onto one of its associated tRNAs an unconventional amino acid or an amino acid other than said given amino acid, and **in that** the nucleic acid sequence of the gene encoding said aminoacyl-tRNA synthetase comprises at least one mutation compared with the sequence of the corresponding wild-type gene, said mutation not having been introduced by a technique of genetic recombination.

19. Cell according to claim 17 or 18, **characterized in that** it is a prokaryotic or eukaryotic cell.

20. Cell according to claim 19, **characterized in that** it is a prokaryotic cell.

21. Cell according to one of claims 17 to 20, **characterized in that** it is chosen from the following cells deposited at the National Microorganism Culture Collection (CNCM), Paris, France:
a) *E coli* strain deposited at CNCM under No. I-2025 on May 25, 1998,
b) *E. coli* strain deposited at CNCM under No. I-2026 on May 25, 1998, and
c) *E. coli* strain deposited at CNCM under No. I-2027 on May 25, 1998,
d) *E. coli* strain deposited at CNCM under No. I-2339 on October 26, 1999,
e) *E. coli* strain deposited at CNCM under No. 1-2340 on October 26, 1999, and
f) *E. coli* strain deposited at CNCM under No. I-2341 on October 26, 1999.

22. Use of a method according to one of claims 1 to 16 for producing a protein, the amino acid sequence of which comprises at least one unconventional amino acid.

23. Use of a cell according to one of claims 17 to 21 for producing a protein, the amino acid sequence of which comprises at least one unconventional amino acid.

24. Process for producing a protein the amino acid sequence of which comprises at least one unconventional amino acid, **characterized in that** it comprises the following steps:
a) where appropriate, selecting a cell by a method according to one of claims 12 to 16;
b) culturing said cell selected in step a) or a cell according to one of claims 17 to 21 in a culture medium and under culture conditions which allow the growth of said cell; and
c) isolating said protein comprising at least one unconventional amino acid from the culture supernatant and/or from the cell pellet obtained in step b).

25. Process according to claim 24, **characterized in that** said culture medium of step b) which allows the growth of said cell contains said unconventional amino acid or one of its precursors.

26. Process according to claim 24, **characterized in that** said unconventional amino acid is synthesized by said cell.

27. Process according to claim 26, **characterized in that** said unconventional amino acid is increased by genetic modification of said cell.

28. Process according to one of claims 24 to 27, **characterized in that** said cell is auxotrophic for the amino acid encoded by said target codon.

29. Process according to one of claims 24 to 28, **characterized in that** said cell comprises a homologous or heterologous gene of interest the coding sequence of which includes at least one target codon.

30. Process according to claim 29, **characterized in that** step b) comprises the compounds required for inducing the synthesis of the protein encoded by said gene of interest.

31. Process according to claim 29 or 30, **characterized in that** biological activity of the protein encoded by said gene of interest is at least partially conserved after the incorporation of said unconventional amino acid at the level of the target codon of said gene of interest.

32. Process according to one of claims 24 to 31, **characterized in that** the unconventional amino acid is chosen from the unconventional amino acids of formula I of configuration L. 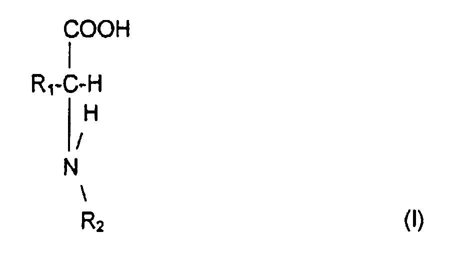 in which:
R₁ or R₂ represents radicals containing a functional group capable of reacting selectively with a chemical or biochemical compound.

33. Process according to claim 32, **characterized in that** the functional group is chosen from the aldehyde, ketone, ethenyl, ethynyl and nitrile groups.

34. Process according to one of claims 24 to 33, for the functionalization of a protein.

35. Process for protein purification, **characterized in that** it comprises the following steps:
a) incorporating into the amino acid sequence of said protein an unconventional amino acid containing a functional group capable of reacting selectively, using a method according to one of claims 24 to 34;
b) bringing the solution containing the protein obtained in step a) into contact with a support comprising a compound capable of reacting specifically with said functional group and of attaching specifically said protein; and
c) isolating said protein attached to the support.

36. Process for attaching a protein to a chemical or biochemical compound, **characterized in that** it comprises the following steps:
a) incorporating into the amino acid sequence of said protein, by a process according to one of claims 24 to 34, an unconventional amino acid containing a functional group capable of reacting selectively;
b) bringing the solution containing the protein obtained in step a) into contact with said chemical or biochemical compound comprising a group capable of reacting specifically with said functional group in a medium allowing the reaction.

37. Process according to claim 36, **characterized in that** said chemical or biochemical compound is itself attached to a solid support or is a constituent compound of a solid support.

38. Process according to claim 36 for preparing a protein complex.

39. Process according to claim 38, **characterized in that** the attached protein or the chemical or biochemical compound is chosen from therapeutic, cosmetic or diagnostic compounds.

40. Process according to claim 38 or 39, **characterized in that** the chemical or biochemical compound is chosen from compounds capable of modifying the biological activity of the attached protein.

41. Process according to claim 38 or 39, **characterized in that** the chemical or biochemical compound is chosen from compounds the biological activity of which can be modified by the attached protein.

42. Process according to one of claims 38 to 41, **characterized in that** the chemical or biochemical compound is chosen from compounds comprising a protein, a polynucleotide, a fatty acid, a sugar or a natural or synthetic polymer.

## Patentansprüche

1. Verfahren, das den Zellen, die ein Gen enthalten, das für ein Protein Pₒ codiert, das für das Wachstum dieser Zellen erforderlich ist, erlaubt, die Fähigkeit zur Herstellung eines Proteins zu erwerben, dessen Aminosäuresequenz mindestens eine nicht herkömmliche Aminosäure AA₁ aufweist, **dadurch gekennzeichnet, dass** es die folgenden Schritte umfasst:
a) das Einführen einer Missense-Mutation an einem für eine Aminosäure AAₒ codierenden Zielcodon in das Gen, das für ein Protein Pₒ codiert, welches für das Wachstum dieser Zellen erforderlich ist, so dass das mutierte Codon für eine Aminosäure AA₁ codiert, wobei das von dem auf diese Weise mutierten Gen synthetisierte Protein P₁ nicht mehr funktionell ist;
b) optional die Kultur der in Schritt a) erhaltenen Zellen, in welche die Missense-Mutation eingeführt worden ist, in einem Nährmedium, das einen Nährstoff enthält, der den Verlust der Funktionalität des auf diese Weise mutierten Proteins P₁ kompensiert; und
c) die Kultur der in Schritt a) oder in Schritt b) erhaltenen Zellen in einem Nährmedium, das die Aminosäure AAₒ enthält, die vor dem Einführen der Missense-Mutation von dem Zielcodon codiert worden ist.

2. Verfahren nach Anspruch 1, **dadurch gekennzeichnet, dass** Schritt c) der Kultur der Zellen eine Reihe von Kulturen der Zellen in einem Nährmedium umfasst, das die von dem Zielcodon codierte Aminosäure enthält, wobei jede dieser Kulturen der Reihe bis zum Erreichen der stationären Wachstumsphase ausgeführt wird, gefolgt von einem Waschen der erhaltenen Zellen, wobei die Anzahl von Kulturen der Reihe ausreicht, um die Selektion von Mutationen, welche die Unterdrückung der Missense-Mutation des mutierten Gens erhöhen, zu erlauben.

3. Verfahren nach einem der Ansprüche 1 und 2, **dadurch gekennzeichnet, dass** die Missense-Mutation aus den Missense-Mutationen ausgewählt ist, die nur mit einer sehr geringen Häufigkeit spontan revertieren, in der Größenordnung von einem Organismus unter mindestens 10¹⁵.

4. Verfahren nach einem der Ansprüche 1 bis 3, **dadurch gekennzeichnet, dass** die Missense-Mutation ein Zielcodon eines Gens, das für ein Protein codiert, das für das Wachstum der Zelle erforderlich ist, in ein Codon umwandelt, das im Vergleich zum Zielcodon eine Änderung von mindestens zwei Basen, vorzugsweise von drei Basen aufweist.

5. Verfahren nach einem der Ansprüche 1 bis 4, **dadurch gekennzeichnet, dass** das Zielcodon für eine Aminosäure mit geringem sterischen Volumen codiert.

6. Verfahren nach einem der Ansprüche 1 bis 5, **dadurch gekennzeichnet, dass** das Zielcodon für eine amphiphile Aminosäure codiert.

7. Verfahren nach einem der Ansprüche 1 bis 6, **dadurch gekennzeichnet, dass** das Zielcodon für eine Aminosäure codiert, deren sterisches Volumen kleiner oder gleich dem sterischen Volumen der von der Missense-Mutation codierten Aminosäure ist.

8. Verfahren nach einem der Ansprüche 4 bis 7, **dadurch gekennzeichnet, dass** das Zielcodon für Cystein codiert.

9. Verfahren nach einem der Ansprüche 4 bis 8, **dadurch gekennzeichnet, dass** die von der Missense-Mutation codierte Aminosäure Valin oder Isoleucin ist.

10. Verfahren nach einem der Ansprüche 1 bis 9, **dadurch gekennzeichnet, dass** der Schritt a) der Transformation der Zellen mittels eines Vektors ausgeführt wird, der eine Sequenz des Gens umfasst, das für ein Protein codiert, welches für das Wachstum der Zellen erforderlich ist, die die Missense-Mutation enthält.

11. Verfahren nach Anspruch 10, **dadurch gekennzeichnet, dass** der Vektor ein Plasmidvektor ist.

12. Verfahren zur Selektion von Zellen, die dazu fähig sind, ein Protein herzustellen, dessen Aminosäuresequenz mindestens eine nicht herkömmliche Aminosäure enthält, **dadurch gekennzeichnet, dass** es die Schritte a), gegebenenfalls b) und c) eines Verfahrens nach einem der Ansprüche 1 bis 12 sowie die Selektion der zum Wachstum befähigten Zellen in Schritt c) umfasst.

13. Verfahren zur Selektion von Zellen nach Anspruch 12, **dadurch gekennzeichnet, dass** es ferner einen Schritt d) der Kultur der in Schritt c) erhaltenen Zellen in einem Nährmedium, das die von dem Zielcodon codierte Aminosäure enthält, wobei die Konzentration der Aminosäure eine höhere Konzentration als jene der in Schritt c) verwendeten Aminosäure sein kann, sowie die Auswahl der Zellen, die für die Konzentration der in Schritt d) verwendeten Aminosäure sensibel sind, umfasst.

14. Verfahren zur Selektion von Zellen nach einem der Ansprüche 12 und 13, **dadurch gekennzeichnet, dass** die Aminoacyl-tRNA-Synthetase, welche die Aminosäure erkennt, die von der Missense-Mutation der selektionierten Zellen codiert worden ist, dazu fähig ist, eine ihrer zugehörigen tRNAs mit einer nicht herkömmlichen Aminosäure oder einer anderen Aminosäure als die von der Missense-Mutation codierten Aminosäure zu beladen.

15. Verfahren zur Selektion von Zellen nach Anspruch 14, **dadurch gekennzeichnet, dass** die Nukleinsäuresequenz des Gens, das für die Aminoacyl-tRNA-Synthetase codiert, im Vergleich zu der Sequenz des entsprechenden Wildtyp-Gens mindestens eine Mutation aufweist.

16. Verfahren zur Selektion von Zellen nach Anspruch 15, **dadurch gekennzeichnet, dass** die Mutation nicht durch eine genetische Rekombinationstechnik eingeführt worden ist.

17. Zelle, die dazu geeignet ist, mittels eines Verfahrens nach einem der Ansprüche 12 bis 16 erhalten zu werden.

18. Isolierte Zelle, die dazu fähig ist, ein Protein herzustellen, dessen Aminosäuresequenz mindestens eine nicht herkömmliche Aminosäure enthält, **dadurch gekennzeichnet, dass** sie eine Aminoacyl-tRNA-Synthetase enthält, die eine gegebene Aminosäure erkennt, welche dazu fähig ist, eine ihrer zugehörigen tRNAs mit einer nicht herkömmlichen Aminosäure oder einer anderen Aminosäure als der gegebenen Aminosäure zu beladen, und dass die Nukleinsäuresequenz des Gens, das für die Aminoacyl-tRNA-Synthetase codiert, im Vergleich zu der Sequenz des entsprechenden Wildtyp-Gens mindestens eine Mutation aufweist, wobei die Mutation nicht durch eine genetische Rekombinationstechnik eingeführt worden ist.

19. Zelle nach Anspruch 17 oder 18, **dadurch gekennzeichnet, dass** es sich um eine prokaryotische oder eukaryotische Zelle handelt.

20. Zelle nach Anspruch 19, **dadurch gekennzeichnet, dass** es sich um eine prokaryotische Zelle handelt.

21. Zelle nach einem der Ansprüche 17 bis 20, **dadurch gekennzeichnet, dass** sie aus den folgenden Zellen ausgewählt ist, die bei der CNCM (Collection Nationale de Culture de Microorganismes, Paris, Frankreich) hinterlegt sind:
a) *E. coli* Stamm, am 25. Mai 1998 bei der CNCM unter der Nummer I-2025 hinterlegt,
b) *E. coli* Stamm, am 25. Mai 1998 bei der CNCM unter der Nummer I-2026 hinterlegt,
c) *E. coli* Stamm, am 25. Mai 1998 bei der CNCM unter der Nummer I-2027 hinterlegt,
d) *E. coli* Stamm, am 26. Oktober 1999 bei der CNCM unter der Nummer I-2339 hinterlegt,
e) *E. coli* Stamm, am 26. Oktober 1999 bei der CNCM unter der Nummer I-2340 hinterlegt,
f) *E. coli* Stamm, am 26. Oktober 1999 bei der CNCM unter der Nummer I-2341 hinterlegt.

22. Verwendung eines Verfahrens nach einem der Ansprüche 1 bis 16 zur Herstellung von Protein, dessen Aminosäuresequenz mindestens eine nicht herkömmliche Aminosäure enthält.

23. Verwendung einer Zelle nach einem der Ansprüche 17 bis 21 zur Herstellung von Protein, dessen Aminosäuresequenz mindestens eine nicht herkömmliche Aminosäure enthält.

24. Verfahren zur Herstellung eines Proteins, dessen Aminosäuresequenz mindestens eine nicht herkömmliche Aminosäure enthält, **dadurch gekennzeichnet, dass** es die folgenden Schritte umfasst:
a) gegebenenfalls die Selektion einer Zelle mittels eines Verfahrens nach einem der Ansprüche 12 bis 16;
b) die Kultur der in Schritt a) selektierten Zelle oder einer Zelle nach einem der Ansprüche 17 bis 21 in einem Nährmedium und unter Kultivierungsbedingungen, die das Wachstum dieser Zelle erlauben; und
c) die Isolierung des Proteins, das mindestens eine nicht herkömmliche Aminosäure enthält, von dem Zellüberstand und/oder von dem in Schritt b) erhaltenen Zellpellet.

25. Verfahren nach Anspruch 24, **dadurch gekennzeichnet, dass** das Nährmedium von Schritt b), welches das Wachstum der Zelle erlaubt, die nicht herkömmliche Aminosäure oder einen ihrer Vorläufer enthält.

26. Verfahren nach Anspruch 24, **dadurch gekennzeichnet, dass** die nicht herkömmliche Aminosäure von der Zelle synthetisiert wird.

27. Verfahren nach Anspruch 26, **dadurch gekennzeichnet, dass** die Synthese der nicht herkömmlichen Aminosäure durch genetische Modifikation der Zelle verstärkt wird.

28. Verfahren nach einem der Ansprüche 24 bis 27, **dadurch gekennzeichnet, dass** die Zelle für die von dem Zielcodon codierte Aminosäure auxotroph ist.

29. Verfahren nach einem der Ansprüche 24 bis 28, **dadurch gekennzeichnet, dass** die Zelle ein homologes oder heterologes Gen von Interesse umfasst, dessen codierende Sequenz mindestens ein Zielcodon enthält.

30. Verfahren nach Anspruch 29, **dadurch gekennzeichnet, dass** Schritt b) die Verbindungen umfasst, die zur Induktion der Synthese des von dem Gen von Interesse codierten Proteins erforderlich sind.

31. Verfahren nach Anspruch 29 oder 30, **dadurch gekennzeichnet, dass** die biologische Aktivität des von dem Gen von Interesse codierten Proteins nach dem Einbau der nicht herkömmlichen Aminosäure am Zielcodon des Gens von Interesse zumindest teilweise erhalten bleibt.

32. Verfahren nach einem der Ansprüche 24 bis 31, **dadurch gekennzeichnet, dass** die nicht herkömmliche Aminosäure aus den nicht herkömmlichen Aminosäuren nach Formel I und in L- Konfiguration ausgewählt ist 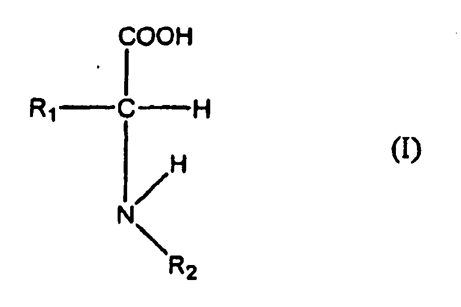 wobei
R₁ bzw. R₂ für Radikale stehen, die eine funktionelle Gruppe enthalten, die dazu fähig ist, mit einer chemischen oder biochemischen Verbindung selektiv zu reagieren.

33. Verfahren nach Anspruch 32, **dadurch gekennzeichnet, dass** die funktionelle Gruppe aus der Aldehydgruppe, Ketongruppe, Vinylgruppe, Ethinylgruppe oder Nitrilgruppe ausgewählt ist.

34. Verfahren nach einem der Ansprüche 24 bis 33 zur Funktionalisierung eines Proteins.

35. Verfahren zur Proteinreinigung, **dadurch gekennzeichnet, dass** es die folgenden Schritte umfasst:
a) den Einbau einer nicht herkömmlichen Aminosäure, die eine funktionelle Gruppe enthält, die dazu fähig ist, selektiv zu reagieren, in die Aminosäuresequenz des Proteins mittels eines Verfahrens nach einem der Ansprüche 24 bis 34;
b) das In-Kontakt-Bringen der Lösung, die das in Schritt a) erhaltene Protein enthält, mit einem Träger, der eine Verbindung umfasst, die dazu fähig ist, mit der funktionellen Gruppe spezifisch zu reagieren und das Protein spezifisch zu fixieren; und
c) die Isolierung des auf dem Träger fixierten Proteins.

36. Verfahren zur Fixierung eines Proteins an einer chemischen oder biochemischen Verbindung, **dadurch gekennzeichnet, dass** es die folgenden Schritte umfasst:
a) den Einbau einer nicht herkömmlichen Aminosäure, die eine funktionelle Gruppe enthält, die dazu fähig ist, selektiv zu reagieren, in die Aminosäuresequenz des Proteins mittels eines Verfahrens nach einem der Ansprüche 24 bis 34;
b) das In-Kontakt-Bringen des in Schritt a) erhaltenen Proteins mit der chemischen oder biochemischen Verbindung, die eine Gruppe enthält, die dazu fähig ist, in einem Medium, das die Reaktion erlaubt, spezifisch mit der funktionellen Gruppe zu reagieren.

37. Verfahren nach Anspruch 36, **dadurch gekennzeichnet, dass** die chemische oder biochemische Verbindung selbst auf einem festen Träger fixiert ist oder eine Verbindung ist, die einen festen Träger bildet.

38. Verfahren nach Anspruch 36 zur Herstellung eines Proteinkomplexes.

39. Verfahren nach Anspruch 38, **dadurch gekennzeichnet, dass** das fixierte Protein bzw. die chemische oder biochemische Verbindung aus den therapeutischen, kosmetischen oder diagnostischen Verbindungen ausgewählt ist.

40. Verfahren nach Anspruch 38 oder 39, **dadurch gekennzeichnet, dass** die chemische oder biochemische Verbindung aus den Verbindungen ausgewählt ist, die dazu fähig sind, die biologische Aktivität des fixierten Proteins zu modifizieren.

41. Verfahren nach Anspruch 38 oder 39, **dadurch gekennzeichnet, dass** die chemische oder biochemische Verbindung aus den Verbindungen ausgewählt ist, deren biologische Aktivität von dem fixierten Protein modifiziert werden kann.

42. Verfahren nach einem der Ansprüche 38 bis 41, **dadurch gekennzeichnet, dass** die chemische oder biochemische Verbindung aus den Verbindungen ausgewählt ist, die ein Protein, ein Polynukleotid, eine Fettsäure, einen Zucker oder ein natürliches oder synthetisches Polymer enthalten.
